# EUROPEAN PATENT APPLICATION

(11) **EP 1 870 462 A1**
(43) Date of publication of application: **26.12.2007**
(21) Application number: 07011328.7
(22) Date of filing: 08.06.2007
(51) Int. Cl.: C12N 15/10

(54) **Method for extracting nucleic acid**

(30) Priority: 09.06.2006 JP 2006161080
(71) Applicant: FUJIFILM Corporation, Minato-ku Tokyo (JP)
(72) Inventor: Kanehara, Hideyuki c/o FUJIFILM Corporation, Ashigarakami-gun, Kanagawa (JP)
(74) Representative: Albrecht, Thomas

(57) **Abstract**

A method for extracting nucleic acid comprises: (a) putting a biological material in contact with a lysing solution to dissolve out nucleic acid; (b) adding a water-soluble organic solvent to an obtained solution of the dissolved nucleic acid, to prepare a lysate solution; (c) putting the lysate solution in contact with a solid material, to allow the nucleic acid to be adsorbed onto the solid material; (d) washing off impurities on the solid material, using a washing solution, wherein twice or more washing procedures are conducted, and a liquid face formed with a washing solution applied in at least one washing procedure other than the first washing procedure among the twice or more washing procedures is higher than a liquid face formed with a washing solution applied in the first washing procedure; and (e) desorbing the nucleic acid adsorbed onto the solid material, using a recovering solution.

## Description

### Background of the Invention

### 1. Field of the Invention

The present invention relates to a method for extracting nucleic acid from biological materials.

### 2. Description of the Related Art

Methods for extracting nucleic acid are broadly divided into methods therefor comprising extracting nucleic acid at a state of solution and methods therefor using solid materials, comprising allowing nucleic acid to be adsorbed onto a solid material by putting a solution containing nucleic acid in contact with the solid material, washing off unnecessary matters and subsequently desorbing the intended nucleic acid from the solid material.

The methods therefor comprising extracting nucleic acid at a state of solution have been done most traditionally, which employ ethanol for precipitating nucleic acid and tangling the resulting nucleic acid around a glass bar. The methods are very simple but disadvantageously have serious problems in terms of yield and purity. Methods capable of overcoming these problems include the AGPC (acid guanidinium phenol chloroform) method comprising adding guanidine thiocyanate to lyse cells and removing DNA concurrently existing, using phenol under acidic conditions to recover RNA, as reported in P.D.Siebert, A.Chenchik, Nucleic Acids Res., 21, 2019-2020 (1993), and the guanidine-cesium chloride precipitation method utilizing the higher suspension density of RNA than that of DNA. However, these methods have various disadvantages such as the use of hazardous organic compounds such as phenol and chloroform and the necessity of laborious procedures and skillful techniques so as to perform the extraction at high precision.

As a method for improving these disadvantages, a method was developed, comprising lysing cells using chaotropic salts such as guanidine thiocyanate inhibiting the activity of enzymes with a nucleic acid-degrading action such as nuclease, then adding ethanol to the resulting solution to prepare a lysate solution, putting the lysate solution in contact with a solid material such as silica to adsorb nucleic acid and desorbing the nucleic acid after a washing step (R. Boom et al., Journal of Clinical Microbiology, 28, 495-503 (1990)).

As an alternative method, a method for extracting nucleic acid using magnetic silica particles was also developed to improve the reaction efficiency and the washing efficiency. An additional method for extracting nucleic acid using a porous membrane was developed as well, so that highly pure nucleic acid can be obtained in such a short period of time by such simple procedures.

The present inventors also developed a method for separating and purifying nucleic acid, using a very thin porous membrane as a material to adsorb solid matters thereon. The method is simpler and has an excellent separation profile. In case that a vast amount of biological materials was used as a sample, for example in case that a larger number of cells was to be treated, it was found that a longer time was apparently required for various solutions to pass through the porous membrane. The inventors made various investigations in terms of dispersing solutions, particularly Bis-Tris buffer, the types and concentrations of surfactants, the types and concentrations of chaotropic salts, the pipetting and agitation procedures after adding a lysing solution, the agitation procedure after adding water-soluble organic solvents, the number of the addition of water-soluble organic solvents, the homogenization method, and the pore size of the porous membrane. Consequently, the inventors developed a method with improved passability, namely a method capable of treating a larger number of cells (JP-A-2003-128691, WO 2007/037509 and JP-A-2006-211973).

### Summary of the Invention

It is an object of the invention to obtain highly pure nucleic acid by reducing impurities in an extract solution of nucleic acid as much as possible. It is another object of the invention to provide a method capable of reducing the occurrence of clogging during the extraction of nucleic acid by shortening the passing time of a washing solution, and therefore capable of treating a larger volume of samples, for example a larger number of cells owing to the improvement of the passability to shorten the extraction time.

It is an additional object of the invention to provide a method for extracting nucleic acid, using a solid phase which can be produced at a large scale and with a substantially constant separation performance, as well as a nucleic acid extraction unit suitable for carrying out the method. It is a further object of the invention to provide a small apparatus for extracting nucleic acid in a simple and rapid manner, with no use of any specific techniques or laborious procedures or any specific apparatuses.

In accordance with the invention, nucleic acid is separated and purified, by lysing a biological material and putting a nucleic acid fraction contained in the biological material in contact with a solid material for example a porous membrane fixed in a container such as cartridge. So as to reduce impurities in the resulting recovered solution containing the extracted nucleic acid, a washing solution is used. The inventors found that the objects could be attained through the examination of the volume of the washing solution. In other words, the invention comprises the following constitutions.
(1) A method for extracting nucleic acid comprising the following steps:
   (a) a step of putting a biological material in contact with a lysing solution to lyse the biological material to dissolve out nucleic acid;
   (b) a step of adding a water-soluble organic solvent to an obtained solution of the dissolved nucleic acid in the step (a), to prepare a lysate solution;
   (c) a step of putting the lysate solution obtained in the step (b) in contact with a solid material, to allow the nucleic acid in the lysate solution to be adsorbed onto the solid material;
   (d) a washing step of washing off impurities on the solid material except the nucleic acid as an extraction subj ect, using a washing solution, wherein twice or more washing procedures are conducted, and a liquid face formed with a washing solution applied in at least one washing procedure other than the first washing procedure among the twice or more washing procedures is higher than a liquid face formed with a washing solution applied in the first washing procedure; and
   (e) an extraction step of desorbing the nucleic acid adsorbed onto the solidmaterial, using a recovering solution.
(2) The method for extracting nucleic acid as described in (1) above, further comprising dispersing the biological material with a dispersing solution prior to adding the lysing solution to the biological material.
(3) The method for extracting nucleic acid as described in (1) or (2) above,
   wherein the lysing solution in the step (a) contains a chaotropic salt at 0.1 to 10 mol/l.
(4) The method for extracting nucleic acid as described in any of (1) to (3) above,
   wherein the lysing solution in the step (a) contains a water-soluble organic solvent at 50 % by volume or less.
(5) The method for extracting nucleic acid as described in (4) above,
   wherein the water-soluble organic solvent contained in the lysing solution is one of methanol, ethanol, propanol and butanol, or a mixture thereof.
(6) The method for extracting nucleic acid as described in any of (1) to (5) above,
   wherein the lysing solution in the step (a) contains a surfactant at 0.001 to 30 % by mass.
(7) The method for extracting nucleic acid as described in any of (1) to (6) above,
   wherein the lysing solution in the step (a) contains a buffer.
(8) The method for extracting nucleic acid as described in any of (1) to (7) above,
   wherein the lysing solution in the step (a) contains a defoaming agent.
(9) The method for extracting nucleic acid as described in any of (1) to (8) above, further comprising adding a solution containing a surfactant at 0.001 to 30 % by mass after the step (a).
(10) The method for extracting nucleic acid as described in any of (1) to (9) above,
   wherein, in the step (b), the water-soluble organic solvent is added to the lysing solution containing the nucleic acid, so as to adjust a volume of the water-soluble organic solvent to 10 % by volume to 60 % by volume to prepare the lysate solution.
(11) The method for extracting nucleic acid as described in any of (1) to (10) above, further comprising performing a mechanical reciprocal motion after at least one of the steps (a) and (b).
(12) The method for extracting nucleic acid as described in any of (1) to (11) above,
   wherein the solid material has a surface comprising hydroxyl group in the step (c).
(13) The method for extracting nucleic acid as described in any of (1) to (12) above,
   wherein a container in which the solid material is retained in a cartridge is used in the step (c).
(14) The method for extracting nucleic acid as described in any of (1) to (13) above, further comprising injecting the lysate solution into two or more containers in the step (c) for extraction.
(15) The method for extracting nucleic acid as described in any of (1) to (14) above,
   wherein foam in the lysate solution is not put into the cartridge during injecting the lysate solution into the cartridge in the step (c).
(16) The method for extracting nucleic acid as described in any of (1) to (15) above,
   wherein the lysate solution is not deposited on an inner cartridge wall except the inner cartridge wall to be immersed with the lysate solution, during injecting the lysate solution into the cartridge in the step (c).
(17) The method for extracting nucleic acid as described in any of (1) to (16) above,
   wherein two kinds of washing solutions having different concentrations of a water-soluble organic solvent are used as the washing solution in the step (d).
(18) The method for extracting nucleic acid as described in any of (1) to (17) above,
   wherein the washing solution contains a salt in the step (d).
(19) The method for extracting nucleic acid as described in any of (1) to (18) above,
   wherein the salt is sodium chloride.
(20) The method for extracting nucleic acid as described in any of (1) to (19) above,
   wherein the washing solution contains a defoaming agent in the step (d).
(21) The method for extracting nucleic acid as described in any of (1) to (20) above, further comprising a step of putting at least one of the lysate solution, the washing solution and the recovering solution in the step(c), (d) or (e) in contact with a solid material via pressure change or centrifugation.
(22) A kit for carrying out a method for extracting nucleic acid as described in any of (1) to (21) above, comprising at least two of a container, a dispersing solution, a lysing solution, a washing solution, a recovering solution and a solid material for adsorbing nucleic acid thereon.
(23) The method for extracting nucleic acid as described in any of (1) to (21) above,
   wherein, in the step(d), a volume of a washing solution applied in at least one washing procedure other than the first washing procedure among the twice or more washing procedures is larger than a volume of a washing solution applied in the first washing procedure.

### Brief Description of the Drawings

Fig.1 shows the relation between the passing time and the yield when the concentration of added NaCl varies; and
Fig.2 shows the effect of addition of a trace amount of ethanol at a low concentration during RNA extraction.

### Detailed Description of the Invention

The invention encompasses the methods described in Japanese Patent Application Nos. 2005-282130, 2005-028991, 2006-027383, 2006-027495, 2005-249694, 2005-082283, 2005-080040, 2005-059057, 2005-029177, 2005-027918, and 2004-066801.

In case that nucleic acid is to be extracted, using a solid material, nucleic acid is recovered by passing a lysate solution through a solid material, washing the solid material with a washing solution, and desorbing nucleic acid from the solid material with a recovering solution. So as to eliminate the possibility of influencing the following steps such as gene detection as much as possible, extracted nucleic acid is preferably at a high purity. For example, chaotropic salts such as guanidine thiocyanate contained in the lysing solution affect enzyme reactions and the like. As a method for reducing various such impurities derived from samples and lysing solutions as much as possible, the inventors focused their attention to and made investigations about a washing process with a washing solution. Consequently, the invention has been achieved.

The method for extracting nucleic acid in accordance with the invention comprises at least the following steps (a) through (e):
(a) a step of putting a biological material in contact with a lysing solution to lyse the biological material and dissolve out nucleic acid contained in the biological material (referred to as "lysis step" hereinafter);
(b) a step of adding a water-soluble organic solvent to prepare a lysate solution (referred to as "lysate step" hereinafter);
(c) a step of putting the lysate solution in contact with a solidmaterial to allow the nucleic acid in the lysate solution to be adsorbed onto the solid material (referred to as "adsorption step" hereinafter);
(d) a washing step of washing the solid material with a washing solution (referred to as "washing step" hereinafter); and
(e) a step of desorbing nucleic acid from the solidmaterial, using a recovering solution and then discharging the nucleic acid out of a cartridge container (referred to as "recovering step" hereinafter).

### 1. Lysis step (a)

By the method for extracting nucleic acid in accordance with the invention, preferably, a biological material is preliminarily dispersed in an appropriate dispersing solution when nucleic acid is to be extracted from the biological material at the step (a). In case that a pelletized cell is to be used, in particular, a dispersing solution is preferably used from the standpoint of improving the dispersibility of the palletized cell. By using a dispersing solution, a biological material at a solid state or at a state close to a solid state can be dispersed, to achieve the homogeneity of the lysed biological material when a lysing solution is added, the stability of the yield and the passing time due to the homogenization, and the shortening of the passing times of the lysate solution, a washing solution and a recovering solution.

Any dispersing solution may be used as long as the dispersing solution can disperse cells while making a biological material swell, disrupted and shrink via the difference in permeation pressure as less as possible. Preferable such dispersing solution includes for example buffers. Among buffers, pH buffers for biochemistry, particularly Good buffers are preferable.

The inventors made investigations. Consequently, the inventors showed that among the Good buffers, Bis-Tris buffer [N,N-bis(2-hydroxyethyl)iminotris(hydroxymethyl)methane] could pass through such solid materials in a short period of time, with a low possibility of the occurrence of clogging. Thus, the Bis-Tris buffer can be used preferably. Other than the Bis-Tris buffer, the Good buffers include for example MES [2-(morpholinoethanesulfonic acid)], HEPES {2-[4-(2-hydroxyhethyl)-1-piperazinyl]ethanesulfonic acid}, PIPES [piperazine-1,4-bis(2-ethanesulfonic acid)], ACES [N-(2-acetamino)-2-aminoethanesulfonic acid], CAPS (N-Cyclohexyl-3-aminopropanesulfonic acid) and TES [N-Tris(hydroxymethyl)methyl-2-aminoethanesulfonic acid].

The volume of the dispersing solution is with no specific limitation. Because a biological material is at a larger volume as the number of cells is larger, nonetheless, the volume of the dispersing solution is increased and used, preferably. However, the increase of the volume of the dispersing solution induces the decrease of the concentration of a chaotropic salt with an action of lysing cells and an action of suppressing the nuclease activity, as well as the increase of the passing time due to the increase of the volume. These indicate that the volume of the dispersing solution for use in accordance with the invention is preferably at 80 % by volume or less of the volume of the lysate solution, more preferably at 50 % by volume or less of the volume of the lysate solution, most preferably at 20 % by volume or less of the volume of the lysate solution.

The dispersing solution is preferably used at a concentration such that cells are wholly or partially never decomposed via the action of permeation pressure. A concentration too large or too small is not preferable. Further, the concentration of the dispersing solution affects the passing times of the lysate solution and a washing solution. In case that the Bis-Tris buffer (pH 6.5) is used at a volume of 30 µl as a dispersing solution, a smaller concentration of the dispersing solution makes longer the passing time of the lysate solution or a washing solution. A larger concentration of the dispersing solution makes cells partially lysed, so that nucleic acid or proteins in the cells are dissolved out. Thus, the nucleic acid is decomposed with the action of dissolved nuclease and the like, leading to a possibility of the decrease of the yield of nucleic acid after extraction. When the yield of nucleic acid is decreased, the amount of impurities compared with nucleic acid is increased. This suggests that the concentration of a dispersing solution used by the method for extracting nucleic acid in accordance with the invention is 0.01 mol/l or more to 10 mol/l or less, preferably 0.1 mol/l or more to 1 mol/l or less.

In case that the pelletized cells are to be prepared, PBS used should be eliminated as much as possible, for exchange with the Bis-Tris buffer or for the addition of the Bis-Tris buffer to the pelletized cells, to markedly shorten the passing time of the lysate solution or a washing solution. After the dispersing solution causing a longer passing time, such as PBS are removed, therefore, another dispersing solution may satisfactorily be added to disperse the cells again. Otherwise, a required volume of Bis-Tris buffer may satisfactorily be added while PBS left at an extremely small volume in the cell pellet is left as it is. When the passing time is not problematic, not any dispersing solution is necessarily used. After addition of the dispersing solution, additionally, the cells may efficiently be dispersed by tapping. By pipetting, alternatively, the cells are dispersedmore efficiently. Still additionally, frozen cell pellets are preferably thawed before these treatments, in terms of operability.

In case of cells cultured in a plate, for example Petri dish, such extraction may be possible with no need of using the dispersing solution. Preferably, the culture broth in the Petri dish is preliminarily removed. A lysing solution as it is may satisfactorily be added to the Petri dish after the removal of the culture broth. From the standpoint of the passability, cells are rinsed using washing solutions such as PBS after the removal of the culture broth. Further, impurities from samples in the extracted nucleic acid are decreased, preferably. These may be required because extra-cellular matrices and the like are removed via washing.

By the method for extracting nucleic acid in accordance with the invention, a biological material is lysed at the step (a). Simultaneously, the biological material is put in contact with the lysing solution so as to dissolve out nucleic acid contained in the biological material. Preferably, the lysing solution contains a chaotropic salt. The chaotropic salt includes for example but is not specifically limited to known chaotropic salts, including for example guanidine salts, sodium isocyanate, sodium iodide, potassium iodide, urea, sodium bromide, potassium bromide, calcium bromide, ammonium bromide, sodium perchlorate, sodium thiocyanate, potassium thiocyanate, ammonium isothiocyanate, sodium chloride, potassium chloride, and ammonium chloride. Among them, guanidine salts are preferable. Guanidine salts include for example guanidine chloride, guanidine isothiocyanate, and thiocyanic acid guanidine salt (guanidine thiocyanate). Among them, guanidine chloride and thiocyanic acid guanidine salt are preferable. These salts may be used singly or in combination of plural such salts.

The concentration of a chaotropic salt in the lysing solution may be any concentration with no specific limitation, as long as cells can sufficiently be lysed at the concentration and additionally, the prepared lysate solution and a washing solution can pass at the concentration in a short period of time. When the concentration is too low, the yield of nucleic acid after extraction is greatly reduced although the passing time can be shortened, due to the insufficient lysis of a biological material. When the concentration is too high, alternatively, the chaotropic salt is deposited at low temperature during the storage of the lysing solution. When the yield of nucleic acid after extraction is reduced, the amount of impurities in the extract solution is increased compared with nucleic acid. Thus, the concentration of the chaotropic salt in the lysing solution is preferably 0.1 to 10 mol/l, more preferably 0.5 mol/l to 5 mol/l or less, most preferably 3 mol/l to 4.5 mol/l or less.

The lysing solution preferably contains a nucleic acid stabilizer. Herein, the term "nucleic acid stabilizer" means a reagent capable of making nucleic acid exist stably in a sample. The nucleic acid stabilizer is a reagent capable of making nucleic acid itself exist stably and additionally includes reagents functioning to prevent the unstable modification for example decomposition of nucleic acid, by reducing or completely inhibiting the nucleic acid-degrading action of nucleic acid-degrading enzymes such as nuclease. Preferably, the nucleic acid stabilizer exists concurrently with any one or more selected from chaotropic salts, surfactants, buffers and defoaming agents.

As the nucleic acid stabilizer with an action to inactivate the nuclease activity, compounds for general use as a reducing agent may be used. Such reducing agent includes for example hydrogen, hydrogenating compounds such as hydrogen iodide, hydrogen sulfide, lithium aluminum hydride, and sodium boron hydride; metals with large electric positivity, such as alkali metals, magnesium, calcium, aluminium and zinc or amalgams thereof; organic oxides of aldehydes, sugars, formic acid and oxalic acid; and mercapto compounds. Among them, mercapto compounds are preferable. The mercapto compounds include for example N-acetylcysteine, mercaptoethanol and alkyl mercaptan. The mercapto compounds may be used singly or in combination of plural such mercapto compounds. In case that 2-mercaptoethanol is used as a nucleic acid stabilizer, the passing time is more shortened as the concentration thereof is higher. However, the higher concentration deteriorates the working environment.

Thus, the concentration of the nucleic acid stabilizer is preferably 0.01 to 20% by mass, more preferably 0.03 to 15 % by mass. In case that the mercapto compound is used as a nucleic acid stabilizer, the concentration of the mercapto compound in the lysing solution is preferably 0.01 to 20 % by mass, more preferably 0.05 to 15 % by mass and most preferably 0.05 to 5 % by mass. (In this specification, mass ratio is equal to weight ratio.)

The inventors found that the passing time of the lysate solution and the passing time of a washing solution could be shortened by adding a surfactant to the lysing solution. Further, a surfactant can highly prevent the decrease of the nucleic acid yield due to the use of a biological material with a smaller number of cells. This is significant, particularly when the concentration of a water-soluble organic solvent in the lysate solution is low. As such example, the concentration of a water-soluble organic solvent may sometimes be reduced so as to shorten the passing time of the lysate solution or a washing solution. In that case, the yield may be decreased although the pasting time can be shortened. When the yield is decreased, the amount of impurities in the extract solution is increased, compared with nucleic acid therein, disadvantageously causing potential troubles in using the resulting nucleic acid after extraction at genetic examinations. Therefore, the addition of a surfactant can improve the decreased yield. The surfactant includes for example nonionic surfactants, cationic surfactants, anionic surfactants, and amphoteric surfactants. Nonionic surfactants and cationic surfactants are preferable.

The nonionic surfactants include polyoxyethylene alkyl phenyl ether-series surfactants, polyoxyethylene alkyl ether-series surfactants and fatty acid alkanol amide. Polyoxyethylene alkyl ether-series surfactants are more preferable. POE decyl ether, POE lauryl ether, POE tridecyl ether, POE alkylene decyl ether, POE sorbitan monolaurate, POE sorbitan monooleate, POE sorbitan monostearate, tetraoleic acid polyoxyethylene sorbit, POE alkylamine, and POE acetylene glycol are more preferable among the polyoxyethylene (POE) alkyl ether-series surfactants.

The cationic surfactants include cetyl trimethylammonium bromide, dodecyl trimethylammonium chloride, tetradecyltrimethylammonium chloride, and cetyl pyridinium chloride.

The inventors made investigations about surfactant concentrations. Consequently, the inventors found that higher surfactant concentrations shortened the passing time of the lysate solution and the passing time of a washing solution. When the surfactant concentration is too high, the amount of the recovered nucleic acid is decreased, depending on the type of a chaotropic salt, together with the occurrence of foaming. By the method for extracting nucleic acid in accordance with the invention, thus, the concentration of a surfactant in the lysing solution is preferably 0.05 % by mass to 30 % by mass, more preferably 0.1 % by mass to 7.5 % by mass.

By using a surfactant, additionally, the lysate solution readily foams. In terms of ready operation, thus, not any surfactant is necessarily used as long as a sufficient performance can be obtained with absolutely no use of any surfactant. Otherwise, a defoaming agent together with a surfactant may be used. Via the use of a defoaming agent, foaming can be suppressed during the preparation of a lysed cell solution or the lysate solution, leading to the improvement of the operability. Further, the suppression of foaming can expectedly lead to the improvement of agitation efficiency, to shorten the passing time of the lysate solution, a washing solution or a recovering solution and additionally to increase the yield. Further, the reduction of the amount of foam introduced during the addition of the lysate solution into the cartridge suppresses the decrease of the purity of the recovering solution due to the remaining foam in the cartridge, to obtain nucleic acid at high purity.

The defoaming agent is effective even when no surfactant is used. In other words, a biological material may sometimes be a source for generating foam, even when no surfactant is contained. By using a defoaming agent, the possibility of the emergence of foam is reduced.

The defoaming agent includes for example silicone-series defoaming agents (for example, silicone oil, dimethylpolysiloxane, silicone emulsion, modified polysiloxane, and silicone compound), alcohol-series defoaming agents (for example, acetylene glycol, heptanol, ethylhexanol, higher alcohol, and polyoxyalkylene glycol), ether-series defoaming agents (for example heptylcellosolve, nonylcellosolve-3-heptylcarbitol), oils and fats-series defoaming agents (for example, animal and vegetable oils), fatty acid-series defoaming agents (for example, stearic acid, oleic acid, and palmitic acid), metal soap-series defoaming agents (for example, aluminium stearate, and calcium stearate), fatty acid ester-series defoaming agents (for example, natural wax, and tributyl phosphate), phosphate ester-series defoaming agents (for example, sodium octyl phosphate), amine-series defoaming agents (for example, diamylamine), amide-series defoaming agents (for example, stearamide) and other defoaming agents (for example, ferric sulfate and bauxite). These defoaming agents may be used singly or in combination of plural such defoaming agents. A combination of the two components, namely a silicone-series defoaming agent and an alcohol-series defoaming agent is particularly preferably used. By the method for extracting nucleic acid in accordance with the invention, the concentration of such defoaming agent in the lysing solution is preferably 0.1 to 10% by mass.

By the method for extracting nucleic acid in accordance with the invention, a water-soluble organic solvent should be mixed in the lysing solution at the step (a), to shorten the passing time of the lysate solution or a washing solution. Particularly, the effect is significant on the passing time of a washing solution. Furthermore, nucleic acid is more readily eluted from the extractionmembrane at a single elution step. At the step (a), the concentration of a water-soluble organic solvent in the lysing solution is preferably 70 % by volume or less, particularly preferably 50 % by volume or less and most preferably 20 % by volume or less.

The type of the water-soluble organic solvent includes for example acetone, alcohols, and dimethyl formamide. Among them, alcohols are preferable. Alcohols may be primary alcohol, secondary alcohol and tertiary alcohol. Particularly, methanol, ethanol, propanol and isomers thereof, and butanol and isomers thereof may preferably be used. Among them, ethanol is particularly preferable from the standpoints of reducing environmental burdens and toxicity. These water-soluble organic solvents may be used singly or in combination of plural such water-soluble organic solvents.

By the method for extracting nucleic acid in accordance with the invention, not any water-soluble organic solvent may satisfactorily be added as long as the passing rate of the lysate solution or a washing solution is sufficiently large. As such example, a case of treating a small number of cells is listed.

Additionally, a mechanical reciprocal motion is preferably applied after adding the lysing solution at the step (a). The mechanical reciprocal motion includes for example a pipetting procedure. In case of a larger number of cells, pipetting is particularly effective. In terms of operability, preferably, pipetting is carried out, using a pipette containing the lysing solution therein, simultaneously with the addition of the lysing solution.

The lysate solution prepared from a biological material after treatment by the pipetting procedure or the lysate solution prepared by lysing a biological material is preferably treated by an agitation procedure. A longer agitation time shortens the passing time of the lysate solution or the passing time of a washing solution, to increase the amount of recovered nucleic acid to stabilize the amount of recovered nucleic acid. An increase of the amount of recovered nucleic acid reduces the amount of impurities compared with nucleic acid after extraction.

The biological material may be homogenized before and after adding the lysing solution, after preparing the lysate solution or at any of the steps. By the homogenization treatment, ingredients extending the passing times, for example substances causing clogging are pulverized or ground to overcome clogging and shorten the passing times. The homogenization treatment may be done by for example ultrasonic treatment, a treatment with a material with a sharp protrusion, a treatment by high-speed agitation, an extrusion treatment from a microfine gap, and treatments by pipetting, or with beads of glass, stainless steel and zirconia.

The homogenization process includes but is not limited to any processes for general use. One example thereof preferably comprises mixing at 30 to 6000 rpm for one second to 3 minutes with an agitation apparatus. In such manner, the yield of nucleic acid finally extracted can preferably be increased, while the passing times can be shortened. Further, the purity of nucleic acid after extraction can be increased. In case of RNA extraction, for example, genome DNA is cleaved, so that the contamination of genome DNA into the extract solution can markedly be reduced.

### 2. Lysate step (b)

By the method for extracting nucleic acid in accordance with the invention, a water-soluble organic solvent is added to the solution of nucleic acid dissolved out by lysing a biological material as prepared at the step (a), which is then put in contact with a solid material with a nucleic acid-adsorbing potency for example a solid material with hydroxyl group on the surface. By the procedure, nucleic acid in a sample solution is adsorbed onto the organic polymer with hydroxyl group on the surface or is captured and adsorbed onto the filter surface or pore of a porous membrane.

The water-soluble organic solvent preferably includes for example but is not limited to alcohols. Alcohols include any of primary alcohol, secondary alcohol and tertiary alcohol, preferably methanol, ethanol, propanol or isomers thereof, and butanol or isomers thereof. These water-soluble organic solvents may be used singly or in combination of plural such water-soluble organic solvents. Ethanol is a particularly preferable water-soluble organic solvent.

When the concentration of the water-soluble organic solvent is low, the amount of recovered nucleic acid is decreased. This is possibly due to the transfer of nucleic acid to be retained on a membrane toward the side of an eluent solution, while the nucleic acid is never associated with the membrane, so that the amount of the recovered nucleic acid is decreased. When the concentration of the water-soluble organic solvent is high, the solutions for example the lysate solution and a washing solution are readily passable, but the amount of recovered nucleic acid is decreased. When the amount of recovered nucleic acid is decreased, the amount of impurities compared with nucleic acid after extraction is increased, so that it is very hard to obtain highly pure nucleic acid. On a concentration basis when the lysate solution is prepared, the concentration of the water-soluble organic solvent at the step (b) is preferably 10 % by volume to 60 % by volume, particularly preferably 20 % by volume to 40 % by volume.

By the method for extracting nucleic acid in accordance with the invention, at least one mechanical reciprocal motion including for example pipetting procedure or agitation procedure or both is preferably carried out after a water-soluble organic solvent is added at the step (b) to the solution of nucleic acid dissolved out by lysing a biological material as prepared at the step (a) . Theagitation procedure may be done at a single time, satisfactorily. However, agitation may be done twice, where each of samples is agitated separately by a first agitation procedure, while the samples are collectively agitated by a second agitation procedure. Two times of agitation procedures are particularly effective for a larger number of samples. A first agitation and a second agitation are preferably done for 0.1 second or more to 600 seconds or less. From the viewpoint of saving user labors, the first agitation time is shorter, while the second agitation time is longer, preferably.

A larger number of pipetting procedures after the addition of the water-soluble organic solvent more shortens the passing times of the lysate solution and a washing solution, leading to the improvement of the yield of nucleic acid. In case of using a smaller number of cells, only a single agitation procedure or a single pipetting procedure is satisfactory from the standpoint of saving user burdens.

### 3. Adsorption step (c)

By the method for extracting nucleic acid in accordance with the invention, the lysate solution obtained at the step (b) is put in contact with a solid material at the step (c), to allow the nucleic acid in the lysate solution to be adsorbed on the solid material. The solid material for use in accordance with the invention may satisfactorily be a single sheet or a plurality of such sheets. In case of using a plurality of such sheets, the solid materials may be the same solid material or may be different solid materials.

By injecting the prepared lysate solution into two or more cartridges at the step (c), even a sample essentially causing clogging or extending the passing time of the lysate solution or a washing solution when only one cartridge is used can be used for extraction without clogging in a short period of time.

A plural number of such cartridges may be used. In case of using a plural number of such cartridges, pipetting procedures may be done once or more times desirably. This homogenizes the lysate solution to consequently load a constant amount of nucleic acid into the cartridges. Additionally, this makes substances causing clogging uniformly distributed, to reduce the occurrence of clogging via the deviation of the passing times due to any non-uniform distribution. Due to the same reasons, the volumes of the lysate solution to be loaded into the cartridges may preferably be equaled as much as possible.

When the lysate solution is to be added to the cartridges, preferably, the lysate solution is added while avoiding foam to be introduced therein as much as possible. This is done so as to avoid the contamination of impurities from foam in the extract solution. The method for avoiding the introduction of foam as much as possible includes for example the use of a defoaming agent during the lysate preparation to suppress foaming, the elimination of foam by centrifugation after the lysate preparation, the prevention of foam aspiration as much as possible, during the addition of the lysate solution from a container for use in the lysate preparation to a cartridge, using a pipette, the addition of the lysate solution so as to allow the lysate solution to flow on the wall face of the cartridge, the aspiration of foam remaining in the cartridge after the addition of the lysate solution to the cartridge, and the elimination of foam by centrifugation after adding the lysate solution to the cartridge.

At the step (c), preferably, the lysate solution is placed in a cartridge while avoiding the deposition of the lysate solution on the inner face of the cartridge except the inner face thereof immersed with the lysate solution in the cartridge. This is done so as to reduce the contamination of impurities from the lysate solution in the extract solution. One example of the method for placing the lysate solution while avoiding the deposition of the lysate solution on the inner face of the cartridge except the inner face thereof immersed with the lysate solution in the cartridge is a method comprising adding the lysate solution while positioning the lower end of a pipette below the liquid face of the lysate solution as formed after the addition of the lysate solution in the cartridge.

### 4. Washing step (d)

By the method for extracting nucleic acid in accordance with the invention, impurities except the nucleic acid to be extracted are rinsed off, using a washing solution at the step (d). Alcohol is used as a water-soluble organic solvent to be contained in the washing solution. Alcohol includes for example methanol, ethanol, isopropanol, n-propanol, and butanol. Propanol may be isopropanol or n-propanol. Butanol may be linear or branched. These alcohols may be used in combination of plural types thereof. Among them, ethanol is preferably used.

The amount of a water-soluble organic solvent contained in the washing solution is preferably at 5 to 100 % by mass. More preferably, the amount thereof is at 5 to 40 % by mass. Within the range, RNA can be obtained at high purity and a high recovery yield, without the increase of DNA contamination or without the desorption of the intended RNA from the porous membrane.

Meanwhile, a water-soluble salt contained in the washing solution is preferably a halide salt, more preferably a chloride. The water-soluble salt is preferably a monovalent or divalent cation; and more preferably, the water-soluble salt includes alkali metal salts or alkali earth metal salts. Among them, sodium salts and potassium salts are preferable. Most preferably, the water-soluble salt is a sodium salt.

In case that the water-soluble salt is to be contained in the washing solution at the step (d), the concentration thereof is 10 mmol/l or more, while the upper limit thereof is not limited to but is satisfactory within a range without any deterioration of the solubility of impurities. The upper limit is preferably 1 mol/l or less, and more preferably 0.1 mol/l or less. The water-soluble salt is preferably sodium chloride, particularly at 20 mmol/l or more.

The washing solution preferably never contains any chaotropic substance. In such manner, any possibility of the contamination of any chaotropic substance at the recovering step can be reduced. When a chaotropic substance contaminates in the recovering step, frequently, the chaotropic substance inhibits enzyme reactions during RT-PCR or the like Therefore, ideally, the washing solution never contains any chaotropic substance in view of the following enzyme reactions and the like. Additionally because chaotropic substances are corrosive and hazardous, no need of the use of chaotropic substances is so advantageous for experimental individuals in terms of the safety for test procedures.

In case that the volume of the lysate solution is less than the cartridge volume and that the lysate solution is to be applied to the inside of the cartridge at the step (d), the height of the liquid face formed with the washing solution applied to the inside of the cartridge at the washing step may be above the height of the liquid face formed with the lysate solution applied in the inside of the cartridge. A method for raising the height of the liquid face thereof comprises for example adding a washing solution of a volume larger than the volume of the lysate solution employed by a specific protocol. The procedure may be done by a non-automatic method or by a method comprising preliminarily inputting the volume of the washing solution in a nucleic acid extractor, and then adding the washing solution automatically. By a centrifugation procedure, the liquid face of the lysate solution may satisfactorily be lowered.

By adjusting the height of the liquid face formed with the washing solution applied to the inside of the cartridge at the washing step above the height of the liquid face formed with the lysate solution applied in the inside of the cartridge, the lysate solution remaining on the wall face inside the cartridge is rinsed to reduce the possibility of the contamination of ingredients derived from the lysate solution in the recovered (extracted) nucleic acid solution, except the nucleic acid to be extracted. When the liquid face formed with the lysate solution inside the cartridge is higher than the liquid face of the washing solution, the lysate solution flows from the phase of the lysate solution formed in the cartridge via a gravitational action to the bottom of the cartridge, to raise the possibility of the contamination of the lysate solution into the recovering solution. When the height of the liquid face formed with the lysate solution is below the height of the liquid face formed with the washing solution, alternatively, the lysate solution is sufficiently rinsed to reduce the possibility of the contamination of ingredients derived from the lysate solution into a recovering solution.

During the preparation of the lysate solution, occasionally, foaming occurs due to various reasons. The inventors found that the contamination of impurities into the recovered solution after the recovery of nucleic acid could be reduced by avoiding the contamination of the resulting foam into the cartridge as much as possible. When foam remains in the cartridge, foam accumulates via the buoyancy thereof around the liquid face formed with the lysate solution and a washing solution in the cartridge, when these solutions are added. In such circumstances, foam remains on the liquid face formed with the solutions in the cartridge. Even when the lysate solution and a washing solution pass through a solid material in the cartridge via pressurization or centrifugation, foam frequently remains as it is on the inner wall of the cartridge. Impurities contaminate from foam remaining in a recovering solution after the recovering solution is applied to the inside of the cartridge, leading to a possibility of the contamination of impurities in a nucleic acid extract solution. By applying the recovering solution to the cartridge while avoiding the contamination of foam as much as possible, the possibility of the contamination of impurities from the lysate solution in the extract solution can be reduced.

In case that the volume of the lysate solution is approximately equal to the cartridge volume at the step (d), the liquid face formed with a washing solution can be made below the liquid face formed with the lysate solution in the cartridge. In such manner, foam formed on the lysate solution in the cartridge can be accumulated at a lower position of the cartridge, which can raise the rinse efficiency at the following washing step. This is due to the following reason.

Many substances causing foam are contained in the lysate solution. A larger volume of the lysate solution at a high concentration causes a higher possibility of foaming during the addition of a washing solution. After adding the lysate solution to the cartridge and passing the lysate solution through a solidmaterial via pressurization or centrifugation, ingredients derived from the lysate solution still remain. When a washing solution is added at that state, foam highly possibly emerges. When the liquid face formed with a first washing solution is below the liquid face formed with the lysate solution in the cartridge, the liquid face formed with a second washing solution can be above the foam layer formed in the cartridge, even if foam emerges and still remains even after passing the first washing solution via pressurization or centrifugation. By raising the liquid volume, in other words, the possibility of foam disruption or the possibility of allowing foam to be washed away during the passing of the washing solution can be raised. It is suggested that the possibility of the contamination of impurities derived from foam in the extract solution can be reduced, consequently, so that highly pure nucleic acid can be obtained. This is effective for a larger volume of the lysate solution so that it is very difficult to adjust the volume of a washing solution to a higher level of the liquid volume of the lysate solution. Foaming is more prominent at a lower concentration of a water-soluble organic solvent. In case that a water-soluble organic solvent at 30 % by volume or less is to be used as a washing solution, the invention is particularly effective.

When the washing procedure at the step (d) for washing a solid material with nucleic acid adsorbed thereon at the step (c) is done several times using a washing solution by the method for extracting nucleic acid in accordance with the invention, the liquid face of a washing solution used in a washing procedure is set to be higher in a latter washing procedure. That is, a liquid face formed with a washing solution applied in at least one washing procedure other than the first washing procedure among the plurality of washing procedures is higher than the liquid face formed with a washing solution applied in the first washing procedure, and preferably the liquid faces formed with washing solutions applied in the washing procedures other than the first washing procedure among the plurality of washing procedures are higher than the liquid face formed with a washing solution applied in the first washing procedure. More preferably, the volume of a washing solution for use in the latter washing procedure should be increased. That is, a volume of a washing solution applied in at least one washing procedure other than the first washing procedure among the plurality of washing procedures is larger than the volume of a washing solution applied in the first washing procedure, and preferably the volumes of washing solutions applied in the washing procedures other than the first washing procedure among the plurality of washing procedures are larger than the volume of a washing solution applied in the first washing procedure. For example, the volume of the first washing solution may be 500 µl, while the second washing solution may be at a volume of 750 µl and the third washing solution may be at a volume of 750 µl. By such procedures, highly pure nucleic acid can be obtained. By adjusting the liquid face formed with a latter washing solution above the position of foam in the cartridge as formed during the addition of the lysate solution or during an earlier washing procedure when foam still remains on the inner cartridge wall during washing, foam is disrupted or rinsed off when a washing solution passes through a solid material via pressurization or centrifugation, so that the volume of impurities from foam in a recovered solution can be reduced to readily allow the recovery of highly pure nucleic acid.

At the washing step (d), washing solutions composed of various concentrations of a water-soluble organic solvent mayalsobeused. Awater-soluble organic solvent, for example ethanol has a defoaming effect. At the washing step, the defoaming possibility is raised by adding a high concentration of a water-soluble organic solvent once or more to the cartridge, so that the rinse efficiency is improved. In such manner, the contamination of impurities in the extract solution is reduced, to permit ready recovery of highly pure nucleic acid. However, a higher concentration of a water-soluble organic solvent is preferably never used at a washing step immediately before adding a recovering solution to the cartridge. This is due to the reason to reduce the contamination of such water-soluble organic solvent in the extract solution.

The washing solution may contain a defoaming agent. This is why the suppression of foaming during washing can suppress the contamination of impurities derived from foam in the extract solution. However, a washing solution containing a defoaming agent is preferably avoided as a washing solution immediately before adding a recovering solution. This is due to the reason to reduce the contamination of the defoaming agent in the extract solution.

The reduction of the volume of the first washing solution at the washing step (d) effectively shortens the passing time. In case that the volume of a washing solution is preset at a given value throughout the washing step, the passing time required for the first washing solution through a membrane is generally the longest. As the number of washing procedures is increased, the passing time is shorter, so that the reduction of the volume of the first washing solution effectively shortens the overall passing time. When the volume of a liquid waste tank for storing the lysate solution and a washing solution is larger after these solutions pass through a membrane, such solutions may deposit on the top of the cartridge via the jumping up of the liquid waste from the liquid waste tank. The depositing solutions may possibly contaminate in the extract solution when the recovered solution passes through the lower end of the cartridge. Nonetheless, the reduction of the volume of the first washing solution reduces the whole liquid volume, so that the jumping up of the liquid waste can be reduced, to obtain more highly pure nucleic acid.

At the washing step (d), a washing solution composed of two different concentrations of a water-soluble organic solventmaybeused. Ahigherconcentrationofawater-soluble organic solvent in a washing solution shortens the passing time of the washing solution through a membrane. In case that RNA is to be extracted, for example, the use of such higher concentration of a water-soluble organic solvent never desorbs genome DNA from a membrane to cause a problem of the contamination of genome DNA into the extract solution. By using a washing solution composed of a water-soluble organic solvent at a lower concentration than that of the washing solution at the first stage, an intermediate stage or the final stage of the washing step, genome DNA can be desorbed from the membrane, to reduce the possibility of the contamination of genome DNA in the extract solution.

In this case, the volume of a washing solution composed of a lower concentration of a water-soluble organic solvent is less than the volume of a washing solution composed of a higher concentration of a water-soluble organic solvent. This is due to the longer membrane-passing time of a washing solution composed of a lower concentration of a water-soluble organic solvent. Additionally, a washing solution at a high concentration of a salt, for example an aqueous sodium chloride solution, namely a washing solution totally without any water-soluble organic solvent can be added intermediately during the washing step, instead of a lower concentration of a water-soluble organic solvent. It is suggested that a high concentration of a salt may promote the adsorption of nucleic acid onto a membrane. The amount of nucleic acid desorbed from the membrane is less even when the washing solution never contains ethanol.

For separating and purifying selectively RNA alone from a lysate solution containing DNA and RNA, the lysate solution passes through a cartridge for nucleic acid extraction, which contains a porous membrane capable of adsorbing nucleic acid, to allow nucleic acid to be adsorbed on the porous membrane capable of adsorbing nucleic acid. Then, washing is done. Subsequently, steps with DNase and the like follow. The type of DNase includes but is not limited to any DNase. In place of DNase, further, a solution containing a water-soluble organic solvent for example ethanol at 50 % by volume or less, preferably 20 % by volume or less, and additionally a salt, for example sodium chloride of preferably 10 mM or more, more preferably 300 mM or more, can be used.

The time required for the step with a DNase action on the porous membrane capable of adsorbing nucleic acid in the cartridge for extracting nucleic acid varies depending on the DNA amount in the solution of nucleic acid mixtures including DNA and RNA and on the concentration of DNase. The time is preferably 5 seconds to 360 minutes, more preferably 30 seconds to 180 minutes. The temperature for the step with DNase on the porous membrane capable of adsorbing nucleic acid in the cartridge for extracting nucleic acid is 4°C or more, preferably 10 to 50°C. So as to raise the reaction efficiency, the step may be done at a high temperature, for example 50 to 70 °C. Herein, the term "with a DNase action on the porous membrane capable of adsorbing nucleic acid in the cartridge for extracting nucleic acid"means an interaction of a site with nucleic acid adsorbed thereon with DNase on the porous membrane capable of adsorbing nucleic acid. The term "on the porous membrane capable of adsorbing nucleic acid" includes not only on the porous membranes capable of adsorbing nucleic acid but also the inside of the pores in the porous membrane and the outlets of the pores on the back face of the membrane. The DNase action effectively shortens the passing time of a washing solution after DNase addition or improves clogging.

Other than DNase, additionally, any one of protese, lipid degrading enzymes, sugar degrading enzymes, nucleases and organic solvents such as chloroform and methanol as well as a mixture thereof may be added. Via the addition thereof, it is suggested that components of substances causing clogging as left on the membrane may be decomposed in an accelerated manner. Consequently, the passability of a washing solution is improved, to shorten the passing time of a washing solution and improve clogging. The addition thereof may satisfactorily be done after passing the lysate solution or may preferably be done after washing several times with a washing solution. In case that protease, lipid degrading enzymes, sugar degrading enzymes and nucleases are to be used, in particular, these degrading enzymes are denatured via the influence of chaotropic salts remaining on the membrane, so that their activities are inhibited, to reduce the potencies thereof to decompose substances causing clogging, potentially leading to no shortening of the passing time of a washing solution. In case of removing genome DNA, further, the activities thereof to cleave genome DNA are reduced.

### 5. Recovering step (e)

By the method for extracting nucleic acid in accordance with the invention, nucleic acid is desorbed from a solid material with a recovering solution and is then discharged from the cartridge container. By adjusting the volume of a recovering solution compared with the volume of a solution of a nucleic acid mixture as prepared from a sample, RNA can be desorbed. The volume of a recovered solution containing RNA separated and purified depends on the volume of a sample used. The volume of a recovering solution for general use is several tens to several hundreds µl. When the sample volume is a trace volume or when it is intended to separate and purify a vast amount of RNA, the volume of a recovering solution varies within a range of 1 µl to several tens milliliters.

As the recovering solution, for example, distilled water and Tris/EDTA buffer may preferably be used. In case that RNA recovered is to be subjected to RT-PCR (reverse transcription polymerase chain reaction) after the step, buffers for use in RT-PCR (in the form of an aqueous solution of KCI, Tris-HCl, MgCl₂, and DTT at final concentrations of for example 75 mmol/1, 50 mmol/1, 3.0 mmol/1, and 10 mmol/l, respectively) may also be used.

The recovering solution is preferably at pH 1 to 10, more preferably pH 2 to 7. Particularly, the ionic strength and the salt concentration have an effect on the dissolution of the adsorbed RNA. The recovering solution is preferably at an ionic strength of 500 mmol/l or less. The salt concentration is preferably 0.5mol/l or less, more preferably 0.01 mmol/l or more to 50 mmol/l or less. In such manner, the recovery ratio of RNA can be improved, so that RNA at a higher yield can be recovered.

By reducing the volume of a recovering solution, a nucleic acid-containing solution in concentration can be recovered. Preferably, the ratio of the volume of a recovering solution to the volume of a solution containing a nucleic acid mixture is preferably 1:100 to 99:100, more preferably 1:10 to 9:10. In such manner, nucleic acid can readily be concentrated without any concentration procedure at a step following nucleic acid extraction. By these methods, a method for obtaining a nucleic acid solution containing nucleic acid as concentrated more than in a sample can be provided.

In another embodiment, by desorbing nucleic acid through the adjustment of the volume of the recovering solution, a solution containing nucleic acid at a desired concentration can be recovered, to provide a recovered solution containing nucleic acid at an appropriate concentration for carrying out the following steps, for example RT-PCR. Preferably, the ratio of the volume of a recovering solution to the volume of a solution containing a nucleic acid mixture is preferably 1:1 to 50:1, more preferably 1:1 to 5:1. In such manner, laborious works for the adjustment of the concentration after nucleic acid extraction can be saved advantageously. Further, the recovery ratio of nucleic acid from the porous membrane can be increased by using a sufficient volume of a recovering solution.

By modifying the temperature of a recovering solution, depending on the object, nucleic acid can be recovered in a simple manner. By desorbing nucleic acid from the porous membrane by adjusting the temperature of a recovering solution to 0 to 10°C, the functions of nuclease can be suppressed with no addition of some reagents or specific procedures for preventing enzyme decomposition, to prevent the decomposition of nucleic acid to obtain a nucleic acid solution in a simple manner and at a very high efficiency. In case that the temperature of a recovering solution is set at 10 to 35°C, further, nucleic acid can be recovered at ambient temperature, to separate and purify nucleic acid via nucleic acid desorption with no need of laborious steps.

In an additional embodiment, the temperature of a recovering solution is adjusted to a high temperature, for example 35 to 70°C, to desorb nucleic acid from the porous membrane without any laborious procedures in a simple manner at a high recovery ratio.

The number of the injection of the recovering solution is not limited to but is one or a plural numerical figure. Generally, nucleic acid is recovered via one injection, for extracting nucleic acid rapidly in a simple manner. So as to recover a vast amount of nucleic acid, the recovering solution may be injected at plural times.

At the recovering step of nucleic acid in the step (e), the recovered solution of nucleic acid should be modified to a composition usable at the following steps. Separated and purified nucleic acid is applicable to RT-PCR (reverse transcription polymerase chain reaction). In this case, the nucleic acid solution extracted is necessarily diluted with a buffer suitable for RT-PCR. At the recovering step by the present method, the use of a buffer suitable for RT-PCR as a recovering solution enables simple and rapid transfer to the following RT-PCR step.

At the aforementioned recovering step, further, a stabilizer may be added so as to prevent the decomposition of nucleic acid recovered in the recovering solution for nucleic acid. As the stabilizer, antibacterial agents, anti-fungal agents and nucleic acid decomposition-suppressing agents may be added. The nucleic acid decomposition-suppressing agents include inhibitors of nucleases, specifically including EDTA. In an additional embodiment, such stabilizers may be added preliminarily to a recovering container.

When the desorbed nucleic acid is never desorbed without several extraction procedures provided that the immersion time is short, the time of the recovering solution immersed with the membrane is made longer in case of desorbing nucleic acid from the membrane, so that a larger amount of adsorbed nucleic acid can be desorbed once or at a small number of extraction procedures. The inventors made investigations in detail. A sufficient amount of nucleic acid could be obtained when the immersion time in extracting nucleic acid was 0.1 second or more to 600 seconds or less, preferably 10 seconds or more to 30 seconds or less.

When the concentration of a surfactant in a lysing solution is increased, therefore, the passing times of the lysate solution and the washing solution can be shortened. So as to gain the expected yield of nucleic acid, several extraction procedures are needed although the concentration of the recovered nucleic acid is lower. By extending the immersion time of the membrane with nucleic acid adsorbed thereon in the recovering solution, nucleic acid can be recovered at a higher yield.

The sample for use in accordance with the invention may include but is not limited to any biological material containing nucleic acid. In the diagnostic field, for example, subj ects for the method may be body fluids such as whole blood, plasma, serum, urine, feces, semen, and saliva collected as samples, or biological materials such as plants (or parts thereof), animals (or parts thereof), bacteria, viruses, culture cells, or lysed materials thereof or homogenized products thereof.

The culture cells include suspending cells and adhesion cells. The suspending cells mean cells growing and proliferating while suspending in culture broths with no deposition on container walls and include for example HL60, U937, and HeLas3 as typical cell lines. The adhesion cells mean cells growing and proliferating in culture broths while deposited on the bottom and wall of a container and include for example NIH 3T3, HEK293, HeLa, COS, CHO cells as typical cell lines. The animals (or parts thereof) for use as samples in accordance with the invention include animal tissues. For example, all tissues composing individual animals, such as liver, kidney, spleen, brain, heart, lung and thymus collectable during animal autopsy or biopsy may be used as such samples. These samples are preferably treated with aqueous solutions of reagents lysing cellular membrane and nucleus membrane to dissolve out nucleic acid, so-called nucleic acid-solubilizing reagents. In such manner, cellular membrane and nucleus membrane are lysed, to obtain a solution of a nucleic acid mixture where nucleic acid is dispersed in an aqueous solution.

In accordance with the invention, the term "nucleic acid" means any of single-stranded, double-stranded, triple-stranded and quadruple-stranded nucleic acids or a mixture thereof. Further, the term includes no limitation to molecular weight. Additionally, any of DNA, RNA and modified products of DNA and RNA, and mixtures thereof may be satisfactory.

In the invention, a solid phase (solid material) having a hydrophilic group means a solid phase (solid material) in which a material itself constituting the solid phase has a hydrophilic group, or a solid phase (solid material) in which a hydrophilic group is introduced into a material constituting the solid phase (solid material) by a treatment or a coating. The material constituting the solid phase may be an organic material or an inorganic material. For example there may be employed a solid phase of which a constituent material is an organic material having a hydrophilic group, a solid phase in which a hydrophilic group is introduced by treating a solid phase of an organic material without a hydrophilic group, a solid phase in which a hydrophilic group is introduced by coating a solid phase of an organic material without a hydrophilic group, with a material having a hydrophilic group, a solid phase of which a constituent material is an inorganic material having a hydrophilic group, a solid phase in which a hydrophilic group is introduced by treating a solid phase of an inorganic material without a hydrophilic group, or a solid phase in which a hydrophilic group is introduced by coating a solid phase of an inorganic material without a hydrophilic group, with a material having a hydrophilic group.

A solid phase of a material having a hydroxyl group can be a solid phase formed by polyhydroxyethylacrylic acid, polyhydroxyethylmethacrylic acid, polyvinylalcohol, polyvinylpyrrolidone, polyacrylic acid, polymethacrylic acid, polyoxyethylene, acetylcellulose or a mixture of acetylcelluloses with difference acetyl values, and preferably a solid phase of an organic material having a hydroxyl group.

The solid phase of an organic material having a hydroxyl group is preferably of a material having a polysaccharide structure, and more preferably a solid phase of an organic polymer formed by a mixture of acetylcelluloses different in acetyl value. The mixture of acetylcelluloses different in acetyl value is preferably a mixture of triacetylcellulose and diacetylcellulose, a mixture of triacetylcellulose and monoacetylcellulose, a mixture of triacetylcellulose, diacetylcellulose and monoacetylcellulose, or a mixture of diacetylcellulose and monoacetylcellulose, particularly preferably a mixture of triacetylcellulose and diacetylcellulose. A mixing ratio of triacetylcellulose and diacetylcellulose is preferably 99:1 to 1: 99, more preferably 90:10 to 50:50.

A more preferable organic material having a hydroxyl group is a saponified substance of acetylcellulose described in JP-A-2003-128691. A saponified substance of acetylcellulose is obtained by a saponification of a mixture of acetylcelluloses different in acetyl value, and is preferably a saponified substance of a mixture of triacetylcellulose and diacetylcellulose, a saponified substance of a mixture of triacetylcellulose and monoacetylcellulose, a saponified substance of a mixture of triacetylcellulose, diacetylcellulose and monoacetylcellulose, or a saponified substance of a mixture of diacetylcellulose and monoacetylcellulose, more preferably a saponified substance of a mixture of triacetylcellulose and diacetylcellulose. A mixing ratio (mass ratio) of triacetylcellulose and diacetylcellulose is preferably 99:1 to 1: 99, more preferably 90:10 to 50:50. In this case, an amount (density) of hydroxyl groups on the solid phase surface can be controlled by a level of saponification process (saponification rate) . A higher amount (density) of the hydroxyl groups is preferable for increasing the separating efficiency of nucleic acid. For example, in case of an acetylcellulose such as triacetylcellulose, the saponification rate (surface saponification rate) is preferably about 5 % or higher, and more preferably 10 % or higher. Also for increasing the surface area of the organic polymer having a hydroxyl group, it is preferable to saponify a solid phase of acetylcellulose.

A saponification indicates contacting acetylcellulose with a saponifying solution (for example an aqueous solution of sodium hydroxide). Thus, in an ester derivative of cellulose contacted with the saponifying solution, an ester group is hydrolyzed and a hydroxyl group is introduced to regenerate cellulose. The regenerated cellulose thus prepared is different from the original cellulose in a crystalline state and the like. Also the saponification rate can be varied with a saponification process under changes in the concentration of sodium hydroxide and in the process time. The saponification rate can be easily measured for example by NMR, IR or XPS (for example by a decrease of a peak of the carbonyl group).

For introducing a hydrophilic group into the solid phase of an organic material without a hydrophilic group, a graft polymer chain having a hydrophilic group in a polymer chain or in a side chain may be bonded to the solid phase. For bonding a graft polymer chain to the solid phase of the organicmaterial, two methods are available, namely a method of chemically bonding the solid phase with a graft polymer chain and a method of polymerizing a compound having a polymerizable double bond starting from the solid phase thereby forming a graft polymer chain.

In the method of chemically bonding the solid phase with a graft polymer chain, the grafting can be conducted by utilizing a polymer having a functional group, capable of reacting with the solid phase, in a terminal end of the polymer or in a side chain, and causing a chemical reaction of the functional group with a functional group of the solid phase. The functional group capable of reacting with the solid phase is not particularly restricted as long as it is capable of reacting with the functional group of the solid phase, and can be, for example, a silane coupling group such as an alkoxysilane, an isocyanate group, an amino group, a hydroxyl group, a carboxyl group, a sulfonic acid group, a phosphoric acid group, an epoxy group, an allyl group, a methacryloyl group, or an acryloyl group. As the polymer having a reactive functional group in a terminal end of the polymer or in a side chain, particularly useful is a polymer having a trialkoxysilyl group in a terminal end of the polymer, a polymer having an amino group in a terminal end of the polymer, a polymer having a carboxyl group in a terminal end of the polymer, a polymer having an epoxy group in a terminal end of the polymer, or a polymer having an isocyanate group in a terminal end of the polymer. The polymer to be employed in this case can be any polymer having a hydrophilic group involved in the adsorption of nucleic acid, but can be, for example, polyhydroxyethylacrylic acid, polyhydroxyethylmethacrylic acid or a salt thereof; polyvinyl alcohol, polyvinylpyrrolidone, polyacrylic acid, polymethacrylic acid or a salt thereof; or polyoxyethylene.

The method of polymerizing a compound having a polymerizable double bond starting from the solid phase thereby forming a graft polymer chain is generally called a surface graft polymerization. The surface graft polymerization means a method of providing a surface of a base material with active species by a plasma irradiation, a light irradiation or a heating, and bonding a compound, having a polymerizable double bond and positioned so as to be contactable with the solid phase, with the solid phase by a polymerization. A compound useful for forming a graft polymer chain bonded to the base material is required to meet two characteristics of having a polymerizable double bond and having a hydrophilic group involved in the adsorption of nucleic acid. Such compound can be, as long as having a double bond within the molecule, a polymer, an oligomer, or a monomer having a hydrophilic group. A particularly useful compound is a monomer having a hydrophilic group. Specific examples of the particularly useful monomer having hydrophilic group include following monomers having a hydroxyl group, such as 2-hydroxyethyl acrylate, 2-hydroxyethyl methacrylate, and glycerol monomethacrylate. Also a carboxyl group-containing monomer such as acrylic acid or methacrylic acid, or an alkali metal salt or an amine salt thereof, can be employed advantageously.

As another method for introducing a hydrophilic group into a solid phase of an organic material not having a hydrophilic group, a material having a hydrophilic group may be coated. A material to be used for coating is not particularly restricted as long as it has a hydrophilic group involved in the adsorption of nucleic acid, but is preferably a polymer of an organic material, in consideration of ease of operation. Such polymer can be, for example, polyhydroxyethylacrylic acid, polyhydroxyethylmethacrylic acid or a salt thereof; polyvinyl alcohol, polyvinylpyrrolidone, polyacrylic acid, polymethacrylic acid or a salt thereof; polyoxyethylene, acetylcellulose or a mixture of acetylcelluloses different in acetyl value, and is preferably a polymer having a polysaccharide structure.

It is also possible, after coating the solid phase of an organic material not having a hydrophilic group with acetylcellulose or a mixture of acetylcelluloses different in acetyl value, to conduct a saponification process on the acetylcellulose or the mixture of acetylcelluloses different in acetyl value thus coated. In such case, the saponification rate is preferably about 5% or higher, more preferably about 10 % or higher.

The solid phase of an inorganic material having a hydroxyl group can be, for example, a solid phase formed by a silica compound or the like. In case of use in a membrane form, it can be a glass filter. It may also be a porous silica film as described in Japanese Patent No. 3058342. Such porous silica film can be prepared by developing, on a base plate, a developing solution of a cationic amphiphilic substance having a bimolecular film-forming ability, then removing a solvent from the liquid film on the base plate thereby preparing a multi-layered film of bimolecular films of the amphiphilic substance, then contacting the multi-layered film of bimolecular films with a solution containing a silica compound and extracting the multi-layered film of bimolecular films.

For introducing a hydrophilic group into the solid phase of an inorganic material without a hydrophilic group, two methods are available, namely a method of chemically bonding the solid phase with a graft polymer chain and a method of polymerizing a monomer, having a hydrophilic group and a polymerizable double bond within the molecule, starting from the solid phase thereby forming a graft polymer chain. In case of chemically bonding the solid phase with a graft polymer chain, a functional group capable reacting with a functional group at a terminal end of the graft polymer chain is introduced into an inorganic material, and a graft polymer is chemically bonded thereto. Also in case of polymerizing a monomer, having a hydrophilic group and a polymerizable double bond within the molecule, starting from the solid phase thereby forming a graft polymer chain, a functional group, serving as a starting point for the polymerization of the compound having the double bond, is introduced into the inorganic material.

The graft polymer having a hydrophilic group and the monomer having a hydrophilic group and a polymerizable double bond within the molecule can preferably be the graft polymer having the hydrophilic group and the monomer having the hydrophilic group and the polymerizable double bond within the molecule, described above in the method of chemically bonding the solid phase of an organic material without a hydrophilic group and a graft polymer chain.

As another method for introducing a hydrophilic group into a solid phase of an inorganic material not having a hydrophilic group, a material having a hydrophilic group may be coated. A material to be used for coating is not particularly restricted as long as it has a hydrophilic group involved in the adsorption of nucleic acid, but is preferably a polymer of an organic material, in consideration of ease of operation. Such polymer can be, for example, polyhydroxyethylacrylic acid, polyhydroxyethylmethacrylic acid or a salt thereof; polyvinyl alcohol, polyvinylpyrrolidone, polyacrylic acid, polymethacrylic acid or a salt thereof; polyoxyethylene, acetylcellulose or a mixture of acetylcelluloses different in acetyl value.

It is also possible, after coating the solid phase of an inorganic material not having a hydrophilic group with acetylcellulose or a mixture of acetylcelluloses different in acetyl value, to conduct a saponification process on the acetylcellulose or the mixture of acetylcelluloses different in acetyl value thus coated. In such case, the saponification rate is preferably about 5 % or higher, more preferably about 10 % or higher.

The solid phase of an inorganic material not having a hydrophilic group may be prepared from a metal such as aluminum, glass, cement, ceramics such as porcelain, new ceramics, silicon or active charcoal.

A nucleic acid-adsorbing porous membrane advantageously employed as the solid phase in the invention is a material through which a solution can pass. A term solution can pass through means that, in case a pressure difference is generated between a space contacting a surface of the membrane and another space contacting the other surface of the membrane, a solution can pass through the interior of the membrane from the space of a higher pressure to the space of a lower pressure. Otherwise, it means that, when a centrifugal force is applied on the membrane, the solution can pass through the interior of the member in a direction of the centrifugal force.

The nucleic acid-adsorbing porous membrane preferably has a thickness of 10 to 500 µm, more preferably 50 to 250 µm. A smaller thickness is preferred in consideration of ease of washing.

The nucleic acid-adsorbing porous membrane may be symmetrical with respect to a front surface and a back surface thereof, but a porous membrane asymmetrical with respect to the front surface and the back surface can be preferably employed.

The nucleic acid-adsorbing porous membrane preferably has a minimum pore size of 0.22 µm or larger, more preferably 0.5 µm or larger. Also a preferred porous membrane has a ratio of a maximum pore size to a minimum pore size of 2 or larger, thereby providing a sufficient surface area for adsorbing nucleic acid and being not easily clogged. More preferably, the ratio of a maximum pore size to a minimum pore size is 5 or larger.

### [Examples]

The invention is now described in detail in the following examples. However, the invention is never limited by them.

### (Example 1)

### Relation between the number of washing procedures with washing solution and the passing time during RNA extraction

30 µl of 0.5M Bis-Tris buffer, pH 6.5 was added to a frozen pellet of HL60 (at 5 ×10⁶ cells) for dispersing the cells via pipetting. 540 µl of LRC (manufactured by Fuji Photo Film Co., Ltd. (now FUJIFILM Corporation)) was added as a lysing solution to lyse the cells, and the resulting mixture was immediately subjected to pipetting five times. Using Cute Mixer CM-1000 (manufactured by EYELA), one-minute agitation was done at 2, 500 rpm, followed by spin down with centrifugation. 260 µl of high grade ethanol (manufactured by Wako Pure Chemical Co., Ltd.) was added for agitation with Cute Mixer CM-1000 at 2,500 rpm for one minute. Subsequently, centrifugation was done for spin down, to prepare a lysate solution.

After a NEXT cartridge (manufactured by Fuji Photo Film, Co. , Ltd.; opening diameter of 7 mm), a washing solution (WRT) and a recovering solution (CRT) were set in QuickGene-800 (manufactured by Fuji Photo Film, Co., Ltd.), the lysate solution was placed in the NEXT cartridge, for extraction by the RNA mode of Quick Gene 800. In that case, presetting was done as follows: the volumes of all the washing solutions were identical at 500 µl; the volume of the recovering solution was 100 µl; and the immersion time in the recovering solution was 120 seconds.

The quantitative determination of recovered RNA and the determination of RNA purity were done, using an ultraviolet and visible spectrophotometer NanoDrop (manufactured by NanoDrop Technologies). The recovery ratio was determined on the basis of the absorbance at 260 nm, while the purity of nucleic acid was determined on the basis of the ratio thereof at 260 nm and 280 nm. When the ratio was 1.8 or more, it was determined that the purity was great. The recovered amount was 386 µg. DNA contamination and the like were analyzed by gel electrophoresis. Conditions for gel electrophoresis were as follows: TAE (Tris-acetate) was used as a buffer; 5 µl of a sample and a loading buffer (10 × Blue Juice) were mixed together, and the resulting whole mixture was electrophoresed.

Consequently, the first passing time of a washing solution was 36.8 seconds; the second, 20.2 seconds; the third, 16.3 seconds; the fourth, 14.9 seconds; and the fifth, 13.2 seconds. The passing time was shortened following the increase of the number of the washing procedures. The passing time means the time from the start of pressurization with the pressurization head of Quick Gene to the time when the solution in the cartridge is passed through the cartridge.

### (Example 2)

### Amount of impurities contained in an extract solution in case of the increase of the volume of a washing solution in the latter washing procedures during RNA extraction

30 µl of 0.5M Bis-Tris buffer, pH 6.5 was added to a frozen pellet of HL60 (at 0.5 ×10⁶ cells) for dispersing the cells via pipetting. 450 µl of a lysing solution containing guanidine thiocyanate as the main ingredient was added to lyse the cells, and the resulting mixture was immediately subjected to pipetting five times. Using Cute Mixer CM-1000 (manufactured by EYELA), one-minute agitation was done at 2,500 rpm, followed by spin down with centrifugation.

195 µl of high grade ethanol was added for agitation with Cute Mixer CM-1000 at 2,500 rpm for one minute. Subsequently, centrifugation was done for spin down, to prepare a lysate solution. After a NEXT cartridge (manufactured by Fuji Photo Film, Co., Ltd. ; opening diameter of 7 mm), a washing solution (WRT) and a recovering solution (CRT) were set in QuickGene-800 (manufactured by Fuji Photo Film, Co., Ltd.), the lysate solution was placed in the NEXT cartridge, for extraction by the RNA mode of Quick Gene 800. In that case, the volume of the lysate solution was 645 µl, while the volume of the first washing solution was 600 µl; the volume of the second washing solution, 750 µl; and the volume of the third washing solution, 750 µl. The volume of a recovering solution was 100 µl, while the immersion time in the recovering solution was preset at 30 second. For comparison, the volumes of all the washing solutions were made equal at 750 µl, for use in extraction.

The quantitative determination of recovered RNA and the determination of RNA purity were done in the same manner as in Example 1. Consequently, the absorbance at 230 nm, mainly the absorbance of guanidine thiocyanate was 1.85 (at an optical path of 1 mm as corrected on a 10-mm basis) when 600 µl of the first washing solution, and 750 µl of the second and third washing solutions were used. When the volumes of the washing solutions were constant at 750 µl in the comparative example, the absorbance at 230 nm was 1.98, indicating that the purity was decreased with the increase of impurities mainly containing guanidine thiocyanate at the constant volume of the washing solutions. Thus, the results described above show that the increase of the volume of the washing solution in the latter procedures causes less impurities contained in the extract solution.

### (Example 3)

### Passing time in case of the increase of the volume of washing solution in the latter stage during RNA extraction

20 µl of PBS was added to a frozen pellet of HL60 (at 5 ×10⁶ cells) for dispersing the cells via tapping. 400 µl of a lysing solution containing guanidine thiocyanate as the main component was added to lyse the cells, and the resulting mixture was immediately subjected to pipetting five times. Using Cute Mixer CM-1000 (manufactured by EYELA), one-minute agitation was done at 2,500 rpm, followed by spin down with centrifugation. 170 µl of high grade ethanol was added for agitation with Cute Mixer CM-1000 at 2, 500 rpm for one minute. Subsequently, centrifugation was done for spin down, to prepare a lysate solution.

After a NEXT cartridge, a washing solution (WRT) and a recovering solution (CRT) were set in QuickGene-800, the lysate solution was placed in the NEXT cartridge, for extraction by the RNA mode of Quick Gene 800. In that case, washing count was preset at 3 times, liquid volume for the first washing was preset at 50 µl, and liquid volumes for the second and third washings were preset at 750 µl. In a Comparative Example, washing count was preset at 3 times, and liquid volumes for the first, second and third washings were preset at 750 µl.

The quantitative determination of recovered RNA and the determination of RNA purity were done in the same manner as in Example 1. Consequently, the RNA yields by the two methods were equal, which were 41 µg. The passing times of the lysate solution by the two methods were equal at about 30 seconds. The passing time of the washing solution 1 (washing solution for the first washing) was 3.7 seconds, the passing time of the washing solution 2 (washing solution for the second washing) was 17.3 seconds, and the passing time of the washing solution 1 in the comparative example was 22.8 seconds. These indicate that the maximum of the passing times of these washing solutions was smaller by 63.2 % in the increase of the washing solution volume in the latter stage than in the comparative example. It is indicated that the increase of the washing solution volume in the latter stage effectively shortened the passing time.

### (Example 4)

### Relation between the volume of washing solution and purity during RNA extraction

30 µl of 0.5M Bis-Tris buffer, pH 6.5 was added to a frozen pellet of HL60 (at 0.5 ×10⁶ cells) for dispersing the cells via pipetting. 450 µl of a lysing solution containing guanidine thiocyanate as the main component was added as a lysing solution to lyse the cells, and the resulting mixture wasimmediatelysubjectedto pipettingfive times. Using Cute Mixer CM-1000 (manufactured by EYELA), one-minute agitation was done at 2, 500 rpm, followed by spin down with centrifugation. 195 µl of high grade ethanol was added for agitation with Cute Mixer CM-1000 at 2, 500 rpm for one minute. Subsequently, centrifugation was done for spin down, to prepare a lysate solution.

After a NEXT cartridge (manufactured by Fuji Photo Film, Co. , Ltd.; opening diameter of 7 mm), a washing solution (WRT) and a recovering solution (CRT) were set in QuickGene-800 (manufactured by Fuji Photo Film, Co., Ltd.), the lysate solution was placed in the NEXT cartridge, for extraction by the RNA mode of Quick Gene 800. In that case, the volume of the lysate solution was 645 µl. In the washing step, washing was conducted for three times with a equal amount of the washing solution for every three times, and the three experiments using 750 µl, 500 µl and 250 µl of the washing solutions for one washing, respectively, were conducted.

The quantitative determination of recovered RNA and the determination of RNA purity were done in the same manner as in Example 1. Consequently, the absorbance at 230 nm, the absorbance of guanidine thiocyanate mainly was 1.98 when a washing solution at 750 µl was used; the absorbance was 2.03 when a washing solution at 500 µl was used; the absorbance was 2.44 when a washing solution at 250 µl was used. Because the volume of the lysate solution was 645 µl, the purity was considerably poor with a 250-µl washing solution volume far smaller than the volume of the lysate solution, while the purity was enhanced as the volume of the washing solution was increased. In case that a washing solution of 750 µl larger than the volume of the lysate solution was used, the purity was at a very great value.

### (Example 5)

### Addition of lysate solution during RNA extraction while avoiding the deposition of the lysate solution onto the wall face of cartridge as much as possible and results

30 µl of 0.5M Bis-Tris buffer, pH 6.5 was added to a frozen pellet of HL60 (at 0.5 ×10⁶ cells) for dispersing the cells via pipetting. 450 µl of a lysing solution containing guanidine thiocyanate as the main component was added as a lysing solution to lyse the cells, and the resulting mixture was immediately subjected to pipetting five times. Using Cute Mixer CM-1000 (manufactured by EYELA), one-minute agitation was done at 2, 500 rpm, followed by spin down with centrifugation. 195 µl of high grade ethanol was added for agitation with Cute Mixer CM-1000 at 2, 500 rpm for one minute. Subsequently, centrifugation was done for spin down, to prepare a lysate solution.

After a NEXT cartridge (manufactured by Fuji Photo Film, Co. , Ltd. ; opening diameter of 7 mm), a washing solution (WRT) and a recovering solution (CRT) were set in QuickGene-800 (manufactured by Fuji Photo Film, Co., Ltd.), the lysate solution was placed in the NEXT cartridge, for extraction by the RNA mode of Quick Gene 800. In that case, the volume of the lysate solution was 645 µl. The volumes of all the washing solutions were equal at 750 µl.

The quantitative determination of recovered RNA and the determination of RNA purity were done in the same manner as in Example 1. Consequently, the absorbance at 230 nm from the lysate solution deliberately deposited on the inner wall of the cartridge was 2.49, while the absorbance from the lysate solution added while avoiding the deposition of the lysate solution onto the inner wall of the cartridge as much as possible was 1.98. This indicates that it is preferable to avoid the deposition of the lysate solution onto the inner wall of the cartridge as much as possible.

### (Example 6)

### Relation between NaCl concentration and the passing time during RNA extraction

20 µl of PBS was added to a frozen pellet of HL60 (at 5×10⁶ cells) for dispersing the cells via tapping. 400 µl of a lysing solution containing guanidine thiocyanate as the main component to lyse the cells, and the resulting mixture was immediately subjected to pipetting five times. Using Cute Mixer CM-1000 (manufactured by EYELA), one-minute agitation was done at 2, 500 rpm, followed by spin down with centrifugation. 170 µl of high grade ethanol was added for agitation with Cute Mixer CM-1000 at 2, 500 rpm for one minute. Subsequently, centrifugation was done for spin down, to prepare a lysate solution.

After a NEXT cartridge, a washing solution (WRT) and a recovering solution (CRT) were set in QuickGene-800, the lysate solution was placed in the NEXT cartridge, for extraction by the RNA mode of Quick Gene 800. In that case, it was presetted that the extractor automatically stops after the lysate solution passes through the extractor, and then washing is conducted for four times (liquid volume for the first washing was 50 µl, and liquid volumes for the second, third and fourth washings were 750 µl). Because the extractor automatically stops after the lysate solution passes through the extractor, 200 µl of 100 to 5000 mM NaCl solutions was added just then. After addition, the extractor was again started.

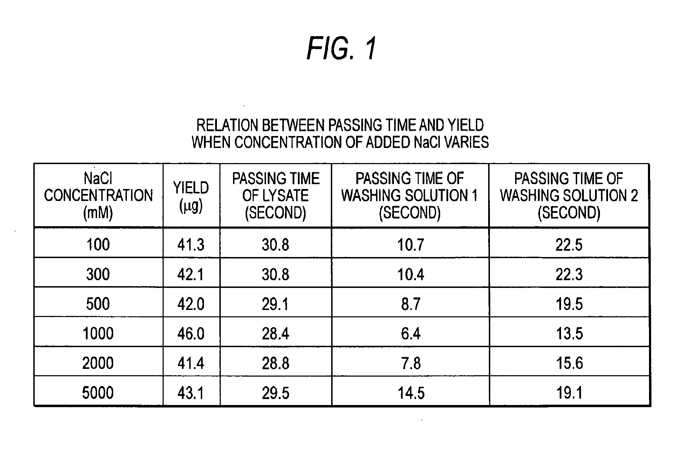

The quantitative determination of recovered RNA and the determination of RNA purity were done in the same manner as in Example 1. Consequently, the relation between the RNA yield and the passing time was identified and is shown in Fig.1. The yield was 41 to 46 µg in all the cases, while the passing times of the lysate solution were almost equal and about 30 seconds in all the cases. At the first washing, the passing time was the shortest at 1, 000 mM NaCl concentration. At the second washing, the passing time was the shortest, namely 13.5 seconds (Fig.1). When NaCI was added at 100 mM, the passing time at the first washing was 10.7 seconds while the time at the second washing was 22.5 seconds. The maximum of the passing times with the first and second washing solutions was shorter by about 40 % at 1000 mM NaCl added than at 100 mM NaCI added. It was shown that the addition of NaCl at 100 mM NaCl was effective for shortening the passing time.

### (Example 7)

### Effect of addition of a trace amount of ethanol at a low concentration during RNA extraction

A liquid culture of HEK 293 cultured in the presence of 5% CO₂ at 37°C for 3 days (2.3 × 10⁶ cells/3.5-cm² dish) was aspirated, and rinsed in 1 ml PBS followed by aspiration. 525 µl of a lysing solution containing guanidine thiocyanate as the main component was added to the dish, to lyse the cells and scrape off the cells with a cell scraper from the dish surface. The resulting solution was transferred into a 1.7-ml microtube. Using Cute Mixer CM-1000 (manufactured by EYELA), one-minute agitation was done at 2,500 rpm, followed by spin down with centrifugation. 175 µl of high grade ethanol was added for agitation with Cute Mixer CM-1000 at 2,500 rpm for one minute. Subsequently, centrifugation was done for spin down, to prepare a lysate solution.

After a NEXT cartridge, a washing solution (WRT) and a recovering solution (CRT) were set in QuickGene-800, the lysate solution was added into the NEXT cartridge, and the added lysate solution passes through the nucleic acid adsorbing membrane contained in the NEXT cartridge under pressure. Next, washing was conducted using a solution mixture containing 50 µl of a washing solution containing 70 % ethanol as the main component and 200 µl of a washing solution containing 10 % ethanol and 500 mM NaCl as the main components, and then washing was conducted for three times using 750 µl of a washing solution containing 70 % ethanol as the main component for one washing.

In a Comparative Example, a washing solution containing 10 % ethanol as the main component was used, and washing was conducted for three times using a liquid volume of 750 µl for one washing.

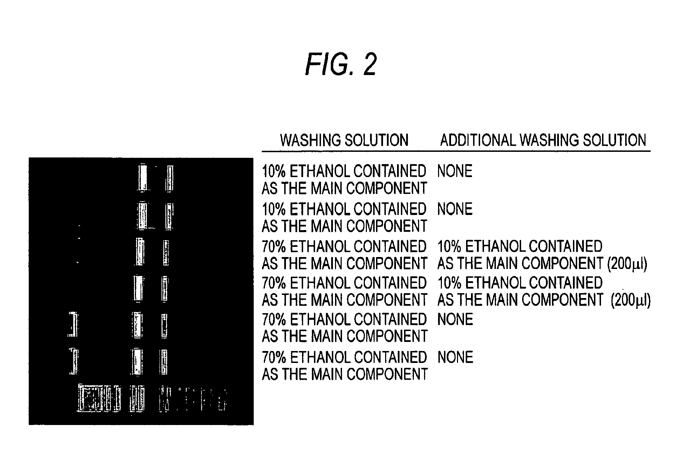

The quantitative determination of recovered RNA and the determination of RNA purity were done in the same manner as in Example 1. Consequently, the passing time of the lysate solution was almost equal to the time in the comparative example, which was about 30 seconds. The passing times of the washing solutions were 4 to 6 seconds, far shorter than in the comparative example, where the passing time was 15 to 25 seconds. In case of no addition of a washing solution containing 10 % ethanol, the contamination due to genome nucleic acid occurred (Fig.2). In case of addition of the washing solution containing 10% ethanol, the passing time of the washing solution was 4 to 6 seconds, and the genome nucleic acid was almost at the same low level as in the comparative example (Fig.2). The yield was about 40 µg in any of the cases. In Fig. 2, the bottom ladder was 1kb Plus DNA Ladder produced by Invitrogen Corporation. Each of the same experiments was conducted twice, and the two results were shown in Fig. 2.

The aforementioned results indicate that the use of smaller volumes of washing solutions at various ethanol concentrations shortens the passing time in RNA extraction, leading to less genome DNA contamination and almost the same RNA yield. Thus, such method is considered as a very effective method.

In accordance with the method of the invention, impurities in the recovered solution after nucleic acid extraction are reduced compared with conventional methods, to obtain the intended product at a higher purity, namely nucleic acid at a higher purity. In accordance with the method of the invention, further, the washing step is modified to shorten the passing time of a washing solution at the washing step and to reduce the frequency of the emergence of clogging, so that a larger number of samples can be treated to improve the through-put of the extraction. In accordance with the method of the invention, still further, nucleic acid can be extracted automatically in a simple and rapid manner, with no need of any specific techniques, laborious procedures and any specific apparatuses.

The entire disclosure of each and every foreign patent application from which the benefit of foreign priority has been claimed in the present application is incorporated herein by reference, as if fully set forth.

## Claims

1. A method for extracting nucleic acid comprising the following steps:
(a) a step of putting a biological material in contact with a lysing solution to lyse the biological material to dissolve out nucleic acid;
(b) a step of adding a water-soluble organic solvent to an obtained solution of the dissolved nucleic acid in the step (a), to prepare a lysate solution;
(c) a step of putting the lysate solution obtained in the step (b) in contact with a solid material, to allow the nucleic acid in the lysate solution to be adsorbed onto the solid material;
(d) a washing step of washing off impurities on the solid material except the nucleic acid as an extraction subject, using a washing solution, wherein twice or more washing procedures are conducted, and a liquid face formed with a washing solution applied in at least one washing procedure other than the first washing procedure among the twice or more washing procedures is higher than a liquid face formed with a washing solution applied in the first washing procedure; and
(e) an extraction step of desorbing the nucleic acid adsorbed onto the solid material, using a recovering solution.

2. The method for extracting nucleic acid according to claim 1, further comprising dispersing the biological material with a dispersing solution prior to adding the lysing solution to the biological material.

3. The method for extracting nucleic acid according to claim 1,
wherein the lysing solution in the step (a) contains a chaotropic salt at 0.1 to 10 mol/1.

4. The method for extracting nucleic acid according to claim 1,
wherein the lysing solution in the step (a) contains a water-soluble organic solvent at 50 % by volume or less.

5. The method for extracting nucleic acid according to claim 4,
wherein the water-soluble organic solvent contained in the lysing solution is one of methanol, ethanol, propanol and butanol, or a mixture thereof.

6. The method for extracting nucleic acid according to claim 1,
wherein the lysing solution in the step (a) contains a surfactant at 0.001 to 30 % by mass.

7. The method for extracting nucleic acid according to claim 1,
wherein the lysing solution in the step (a) contains a buffer.

8. The method for extracting nucleic acid according to claim 1,
wherein the lysing solution in the step (a) contains a defoaming agent.

9. The method for extracting nucleic acid according to claim 1, further comprising adding a solution containing a surfactant at 0.001 to 30 % by mass after the step (a).

10. The method for extracting nucleic acid according to claim 1,
wherein, in the step (b), the water-soluble organic solvent is added to the lysing solution containing the nucleic acid, so as to adjust a volume of the water-soluble organic solvent to 10 % by volume to 60 % by volume to prepare the lysate solution.

11. The method for extracting nucleic acid according to claim 1, further comprising performing a mechanical reciprocal motion after at least one of the steps (a) and (b).

12. The method for extracting nucleic acid according to claim 1,
wherein the solid material has a surface comprising hydroxyl group in the step (c).

13. The method for extracting nucleic acid according to claim 1,
wherein a container in which the solid material is retained in a cartridge is used in the step (c).

14. The method for extracting nucleic acid according to claim 1, further comprising injecting the lysate solution into two or more containers in the step (c) for extraction.

15. The method for extracting nucleic acid according to claim 13,
wherein foam in the lysate solution is not put into the cartridge during injecting the lysate solution into the cartridge in the step (c).

16. The method for extracting nucleic acid according to claim 13,
wherein the lysate solution is not deposited on an inner cartridge wall except the inner cartridge wall to be immersed with the lysate solution, during injecting the lysate solution into the cartridge in the step (c).

17. The method for extracting nucleic acid according to claim 1,
wherein two kinds of washing solutions having different concentrations of a water-soluble organic solvent are used as the washing solution in the step (d).

18. The method for extracting nucleic acid according to claim 1,
wherein the washing solution contains a salt in the step (d) .

19. The method for extracting nucleic acid according to claim 18,
wherein the salt is sodium chloride.

20. The method for extracting nucleic acid according to claim 1,
wherein the washing solution contains a defoaming agent in the step (d).

21. The method for extracting nucleic acid according to claim 1, further comprising a step of putting at least one of the lysate solution, the washing solution and the recovering solution in the step (c), (d) or (e) in contact with a solid material via pressure change or centrifugation.

22. A kit for carrying out a method for extracting nucleic acid according to claim 1, comprising at least two of a container, a dispersing solution, a lysing solution, a washing solution, a recovering solution and a solid material for adsorbing nucleic acid thereon.

23. The method for extracting nucleic acid according to claim 1,
wherein, in the step (d), a volume of a washing solution applied in at least one washing procedure other than the first washing procedure among the twice or more washing procedures is larger than a volume of a washing solution applied in the first washing procedure.
